# EUROPEAN PATENT SPECIFICATION

(11) **EP 2 490 727 B1**
(45) Date of publication and mention of the grant of the patent: **23.09.2020**
(21) Application number: 10765807.2
(22) Date of filing: 19.10.2010
(51) Int. Cl.: A61L 27/26, A61L 27/54

(54) **COMPOSITION FOR THE TREATMENT OF A BONE FRACTURE**
ZUSAMMENSETZUNG ZUR BEHANDLUNG VON KNOCHENBRÜCHEN
COMPOSITION DESTINÉE AU TRAITEMENT D'UNE FRACTURE OSSEUSE

(30) Priority: 23.10.2009 SE 0950783; 23.10.2009 US 254609 P
(43) Date of publication of application: 29.08.2012
(73) Proprietor: Biomedical Bonding AB, 187 63 TÄBY (SE)
(72) Inventor: HULT, Anders, 187 34 Täby (SE); MALKOCH, Michael, 187 63 Täby (SE); VON HOLST, Hans, 182 66 Djursholm (SE); NORDBERG, Axel, 112 31 Stockholm (SE)
(74) Representative: Brann AB
(86) International application number: PCT/EP2010/065689
(87) International publication number: WO 2011/048077

(56) References cited:
- WO-A1-03/011926
- WO-A1-2009/058079
- WO-A2-2005/086911

## Description

### Technical field

The present invention relates to compositions and their use for the treatment and stabilisation of bone fractures.

### Background

Orthopaedic healthcare costs are increasing worldwide. With a growing elderly population and an increasing demand for advanced healthcare globally, new efficient treatments are needed. Bone fractures are often stabilized using conventional screws and plates such as described in Miller D, L Goswami T in Clinical Biomechanics 2007;22:1049-1062.
However, these implants pose a number of drawbacks due to their inflexible forms. Insertion of screws and plates requires open surgery, thus limiting the accessibility to proximate fractures. For fractures that cannot be reached during open surgery, stabilization with screws and plates cannot be performed. An important drawback is also that plates demand drilling and screws to be fixated. Since it may be unsuitable to screw into thin and sensitive bone structures, for example found in cervical spine-, cranio-maxillofacial- and hand-surgery, it may be difficult to place plate implants in-vivo. It is also not always possible to fit a small number of predefined plates on a large number of different fractures. For those fractures located in the cervical spine that cannot be treated with conventional implants, a Halo vest is often used such as described in (Vieweg U, Schulteiss R in Archives of Orthopaedic and trauma surgery 2001;121:50-55). Although conventional methods are effective, problems with small fracture areas, dislocations and patient discomfort are still persistent.

For compression fractures on vertebral bodies, vertebroplasty or kyphoplasty is often used. By inflating the vertebrae with bone filler, e.g. bone cement or a balloon, the fracture is stabilized. The technique is widely used and can be performed with minimally invasive surgery. The need for minimally invasive surgery for vertebral fractures is constantly increasing as they can curb complications such as excessive blood loss, extended recovery period and post-operative trauma caused by conventional screw and plate methods.

Bone fillers have been developed and manufactured by a number of companies, among them Doxa AB and Bone Support AB, both of Sweden. Contrary to positive reports regarding their mechanical stability and flexibility, concerns about infection or cement leakage into the spinal canal or into the perivertebral venous system have surfaced (M.E. Jensen, A.J. Evans, J.M. Mathis, D.F. Kallmes, H.J. Cloft and J.E. Dion in Am. J. Neuroradiol. 18 (1997), pp. 1897-1904). Furthermore, grave complications, such as fatal pulmonary failure in the immediate postoperative period, have been reported on. They were commonly due to thrombotic tissue embolism while embolism caused by the cement itself remained asymptomatic (F. Monticelli, H.J. Meyer, E. Tutsch-Bauer in Forensic Science International, Vol 149,1, (2005) pp 35-38). Reports have also suggested adjacent vertebra fracture caused by imbalance of load distribution after vertebroplasty procedures.

In dental applications various glues have been used as fissure sealants, tissue adhesives, dentine seals and as denture reparative agents(Peter A. LEGGAT, Ureporn KEDJARUNE and Derek Richard SMITH Industrial Health 2004, 42, 207-211). For these purposes, traditionally, acrylic adhesives have been used with acceptable strength for its purpose but with regular reports on toxic response both on cell culture and in-vivo studies. Many experimental adhesives found in literature either possess good biocompatibility or good strength. (Ciapetti G, Stea S, Cenni E, Sudanese A, Marraro D, Toni A, Pizzoferrato A Biomaterials. 1994 Jan;15(1):63-7).

Other commercially available adhesives/fillers for similar bone fracture problems have been described such as chronOS® for Spine fractures by Synthes North America. ChronOS® is a non synthetic, biocompatible bone void filler, and is ideal for many bone void filler applications. The material is a radiopaque β-tricalcium phosphate which contains two of the main mineral constituents of bone, calcium and phosphorus. ChronOS® is resorbed and replaced by bone in 6-18 months during the healing process.

Cryolife's BioGlue® is a surgical adhesive and is composed of purified bovine serum albumin (BSA) and glutaraldehyde. The two glue components are dispensed by a controlled delivery system comprising a double-chambered syringe, applicator tips, and optional extender applicator tips. Once dispensed, the adhesive components are mixed within the applicator tip where the crosslinking begins.

Glutaraldehyde molecules bond with the BSA molecules and, upon application to the tissue proteins at the repair site, create a flexible mechanical seal. It begins to polymerize within 20 to 30 seconds and reaches its bonding strength within two minutes. It also adheres to synthetic graft materials through mechanical bonding within the interstices of the graft material.

Cohera Medical is developing a small bone adhesive that chemically bonds with adjacent bone and resorbs in a timely fashion to allow normal bone healing. Testing of the strength of glued lap joints in porcine bones revealed that the adhesive under development exhibits significant strength and adherence to bone, suggesting that it could be quite useful for fixation of small bones where mechanical fixation is problematic or impossible.

Maurer P, Bekes K, Gernhardt CR, Schaller H-G, Schubert J in International Journal of Oral & Maxillofacial Surgery 2004; 33:377-381 Compares a number of dental adhesives to cortical bone with various results.

Szep S, Kunkel A, Ronge K, Heidemann D in Journal od Biomedical Materials Research: Applied Materials 2002: 53-60 finds a high rate of cell apoptosis in a number of dental adhesives.

Previous studies have showed that reinforcing adhesives with fibres increases mechanical strength on bonded bone fractures. They have shown better fracture stability when compared to using only conventional biomaterial adhesive fillers.

Nordberg A, von Holst H, Brolin K, Beckman A in BioMedical Materials and Engineering 2007;17 (5) :299-308 discloses fibre reinforced adhesives for fixating vertebral fractures.

PCT publication "WO 03/080144 A1" describes a polymeric cement for augmentation of bone fractures. The cement is either applied in a fracture or as filler, in e.g. in a vertebra. However, this method is limited to fractures with even/smooth surfaces, or to voids, such as an osteoporotic vertebra.
PCT publication "WO 2005/027988 A2" describes a calcium salt composition alternatively demineralised bone reinforced with discrete fibres as a bone cement. Although this innovation increases the mechanical properties of calcium salt cements the method is limited to be used as filler of voids e.g. osteoporotic vertebrae and mainly for compressive loads.

PCT publication WO 2009/029734 A2 describes a polymeric bone cement with a several alternative compositions including thiol-ene chemistry. The invention includes a filler that can be chosen to promote bone formation. However, regardless of composition the bone cement and its related method of application is limited to be injected in bone voids e.g. vertebrae and dental applications. PCT publication WO2005/086911 A2 describes dental restorative mixtures wherein the mixture comprises polythiol compounds and one or more polyvinyl compounds.

Problems with the presently available adhesives include too low strength, too low adhesion and too high toxicity. It would be thus desirable to use a biocompatible and mechanical robust adhesive when stabilizing bone fractures. Since adhesives do not demand drilling and can be distributed via minimally invasive surgery it also possesses long term advantages. It is also possible with adhesive fixation to use degradable substances that are naturally resorbed during the natural bone healing process.

### Summary

It is an object of the present invention to obviate at least some of the disadvantages in the prior art and provide an adhesive patch and a kit for the treatment of at least one selected from a bone fracture and a bone cavity, as defined in claims 1 and 8.

In a first aspect there is provided a composition formed by a reaction of at least one component A and at least one component B, wherein component A is selected from the group consisting of a compound comprising at least two thiol-groups and a disulfide derivative of a compound comprising at least two thiol groups, and wherein component B is a compound comprising at least two vinyl groups,for the manufacture of an implant for the treatment of a bone fracture.
In a second aspect there is provided use of the composition for the manufacture of an implant for the treatment of at least one selected from a bone fracture and a bone cavity.
In a third aspect there is provided a method of treating at least one selected from a bone fracture and a bone cavity, said method comprising the steps applying the composition at the site of the fracture in the body.

In a fourth aspect there is provided an implant comprising a fibre and the composition as described above for the treatment of a bone fracture.
In a fifth aspect there is provided a kit for the treatment of at least one selected from a bone fracture and a bone cavity, said kit comprising the composition above and a primer.

Advantages include that the adhesive patch formed by the composition will be solid in body fluid upon curing and will exhibit excellent mechanical strength.
Another advantage is that the material can be applied in small and inaccessible areas. It is an advantage that minimal invasive surgery can be used.
Yet another advantage is that the process is comparable to the application of regular bone fillers and thus requires less surgeon retraining.

Since there is provided the possibility of a minimal invasive surgical procedure, it will further solve drawbacks with open surgery involving drilling and screwing. This would reduce operational time, post operational trauma and recovery and hospitalization costs.
Yet another advantage is that it is possible to use cost effective materials and methods in the process.

Further objects and advantages of the invention will be apparent from the figures and descriptions that follow.

### Detailed description

The invention is defined by the claims. Any subject-matter falling outside the scope of the claims is provided for information purposes only. Any references in the description to methods of treatment or surgery refer to the compounds, pharmaceutical compositions and medicaments of the present invention for use in a method for treatment or surgery of the human (or animal) body by therapy.

It must be noted that, as used in this specification and the appended claims, the singular forms "a", "an" and "the" include plural referents unless the context clearly dictates otherwise.

If nothing else is defined, any terms and scientific terminology used herein are intended to have the meanings commonly understood by those of skill in the art to which this invention pertains.
The term "about" as used in connection with a numerical value throughout the description and the claims denotes an interval of accuracy, familiar and acceptable to a person skilled in the art. Said interval is ± 10 %.
"Compacted fracture" is used herein to denote a fracture caused when bone fragments are driven into each other.

"Complete fracture" is used herein to denote a fracture in which bone fragments separate completely.

"Compression fracture" is used herein to denote a fracture in which bone is compressed.

"Fibre" is used herein to denote a class of material which comprises continuous filaments and/or discrete elongated pieces.

"Incomplete fracture" is used herein to denote a fracture in which the bone fragments are still partially joined.

"Linear fracture" is used herein to denote a fracture that is parallel to the bone's long axis.

"Oblique fracture" is used herein to denote a fracture that is diagonal to a bone's long axis.

"Spiral fracture" is used herein to denote a fracture where at least one part of the bone has been twisted.

"Transverse fracture" is used herein to denote a fracture that is at a right angle to the bone's long axis.

### Brief description of the drawings

The invention is described in greater detail with reference to the drawings in which:
Fig 1a and 1b show embodiments of component A.
Fig 2a and 2b show embodiments of component B.
Fig 3a and 3b show embodiments of a primer.
Fig 4 shows an exploded view of a bone fracture which is repaired according to the present invention.
Fig 5 shows NMR characterization of the compounds poly(maleic anhydride alt methyl vinyl ether)and allyl functionalized hydrophilic poly(maleic anhydride alt methyl vinyl ether)
Fig 6 shows Raman spectroscopy of component A, tris[2-mercaptopropionyloxy)ethyl]isocyanurate, component B, 1,3,5-triallyl-1,3,5-triazine-2,4,6(1*H*,3*H*,5*H*)-trione and cured composition from component A and component B
Fig 7 shows the cytotoxicity of bone adhesives and primers.
Fig 8 shows shear strength of adhesives.

There is provided a composition formed by a reaction of at least one component A and at least one component B, wherein component A is selected from the group consisting of a compound comprising at least two thiol-groups and a disulfide derivative of a compound comprising at least two thiol groups, and wherein component B is a compound comprising at least two vinyl groups, for the manufacture of an implant for the treatment of a bone fracture.

Component A and B can be used in varying molar ratios for optimization purposes, such as adhesion, mechanical strength and crosslinking efficiency. A skilled person can in the light of this description perform routine experiments to optimize the ratio of A and B to obtain the properties. Preferably the ratio of A and B are optimised starting with an equimolar ratio with respect to functionality.

The composition becomes cross-linked after curing. The composition is cured via various methods. These methods include but are not limited to, spontaneous curing, heat induced curing and ultra-violet light induced curing.

Any number of different components A and components B can be used together, such as A1+A2+A3+B1+B2, to form the composition.

In one embodiment component A and component B are mixed without the addition of any solvents. In an alternative embodiment component A and component B are mixed with at least one solvent. Examples of solvents include but are not limited to water and ethanol.

In one embodiment the compound in component A further comprises at least one group selected from the group consisting of a hydroxyl group, a carboxyl group, a dopamine group, and a phenol group.

In one embodiment the component A is a polymer. In such cases the polymer molecular weight is from 1 to 100 kDa. In case of a polymer the substitution degree of thiol groups or disulfide groups on the polymer is from 1% to 100% of all possible substitution sites.

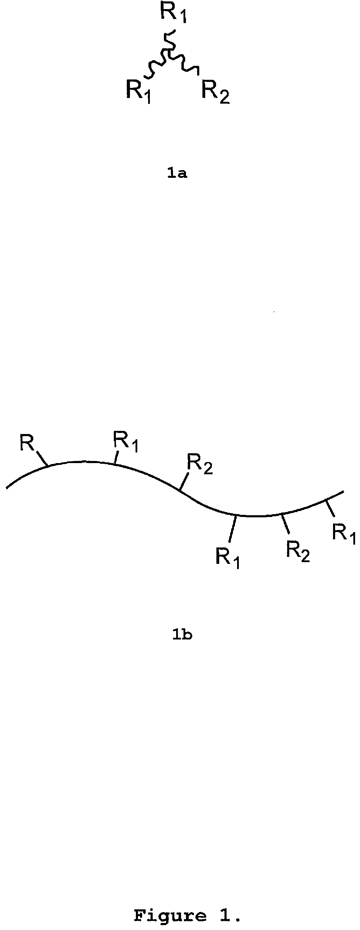

Component A is described further with reference to Fig 1a showing an embodiment of component A with two thiol groups R1 and one other substituent R2, wherein R2 is selected from the group consisting of a hydroxyl, a carboxyl, a dopamine and a phenol group. In an alternative embodiment illustrated in Fig 1b component A is a polymer with several thiol groups and/or disulfide groups R1, and optionally at least one group R2 which is selected from the group consisting of a hydroxyl, a carboxyl, a dopamine and a phenol group.

In one embodiment component A is selected from Pentaerythritol tetrakis(3-mercaptopropionate), trimethylolpropane tris(3-mercaptopropionate), tris[2-mercaptopropionyloxy)ethyl]isocyanurate, Mercaptopropyl methylsiloxane-dimethylsiloxane copolymer, poly(mercaptopropyl)methylsiloxane, 2,2'-(Ethylenedioxy)diethanethiol, Ditiotreitol, tetraethyleneglycol-bis(3-mercaptopropionate), Ethyleneglycol-bis(3-mercaptopropionate), trimethylolpropane diallylether, Dipentaerytritolhexakis(3-merkaptopropionate),tetradecane-1,14-dithiol, (+/-)-trans-1,2-bis(2-mercaptoacetamido)cyclohexane, (E)-S,S'-bis(10-mercaptodecyl)-4,4'-(diazene-1,2-diyl)bis(4-cyanopentanethioate), bis(2-mercaptoethyl)sulfone, 2,5-dimercaptomethyl-1,4-dithiane, 1,4-butanediol-bis(3-mercaptopropionate), 1,16-hexadecanedithiol, undecane-1,11-dithiol, heptane-1,7-dithiol, 1,12-dimercaptododecane, octadecane-1,18-dithiol, (5-mercaptomethyl-2,4-dimethyl-phenyl)-methanethiol, (3-mercaptomethyl-5-methyl-phenyl)-methanethiol, 1,2-benzenedimethanethiol, (4R,5R)-4,5-bis(mercaptomethyl)-2,2-dimethyl-1,3-dioxolane, 3-bis(2-mercaptoethylthio)propane, ethanethiol, aceticacid-mercapto-1,2,6-hexanetriyl ester, L-1,4-dithiothretol, glycerylthioglycolate, 3,6-dioxa-1,8-octanedithiol, trimethylolpropane-tris(mercaptoacetate), 2,3-butanediol-1,4-dimercapto- pentaerythritol-tetrakis(3-mercaptopropionate), ethanethiol-2,2',2"-nitrilotris, 2,2'-thiodiethanethiol, 1,9-nonanedithiol, 2,2'-oxydiethanethiol, and 10-decanedithiol.

In another embodiment component A is synthesized from compounds, including linear and dendritic polymers, with a minimum functionality of two or more. Such as compounds include but are not limited to compounds with reactive groups of isocyanates, alcohols, amines, epoxides, methacrylates, acrylates, carboxylic acids, anhydrides, and allyls.

In another embodiment, component A is obtained by reacting hydrophilic linear polymers, such as PVA1, PHEMA, dendritic materials and maleic anhydride substituted polymers with thiol-groups, or a disulfide derivative thereof, components to give thiol substituted polymers.

In one embodiment component A is selected from the group consisting of
- poly(maleic anhydride - methyl vinyl ether) substituted with 50% cysteamin,
- tris(2-mercaptopropionlyloxy)ethyl isocyanurate, and
- tetraethyleneglycol bis (3-mercaptopropionate).

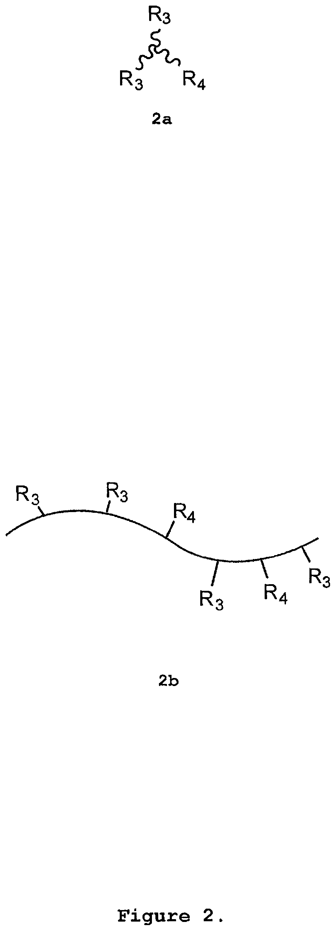

Component B is described referring to Fig 2b which shows an embodiment where component B is a molecule comprising two vinylic functional groups R3 and one group R4 selected from a hydroxyl, a carboxyl, a dopamine, and a phenol group. In an alternative embodiment depicted in Fig 2b component B is a polymer comprising at least two vinylic functional groups R3 and optionally at least one group R4 selected from a hydroxyl, a carboxyl, a dopamine, and a phenol group.

In one embodiment component B is a triazine.

In one embodiment component B comprises vinyl reactive groups selected from vinyl, acrylates, methacrylates, allyls and unstaturated cyclic vinyls including norbornenes and N-maleinimides.

In yet one embodiment component B is selected from the group consisting of trimethylolpropane diallyl ether, 1,3,5-triallyl-1,3,5-triazine-2,4,6(1*H*,3*H*,5*H*)-trione, trimethylolpropane diallyl ether, poly(ethylene glycol) diacrylate, poly(ethylene glycol) dimethacrylate and poly(ethylene glycol) dimaleinimide.

In another embodiment component B is synthesized from compounds, with a minimum functionality of two or more, such as compounds with reactive groups of isocyanates, alcohols, amines, epoxides, carboxylic acids and anhydrides.

In another embodiment other linear or dendritic polymers are used for functionalization with vinyl-groups of component B. Examples include but are not limited to polycaprolactone with di-,tri, and tetra hydroxyl functionality and 2,2-bis(hydroxymethyl)propanoic acid based dendritic materials.

In yet one embodiment, component B is obtained by reacting hydrophilic linear polymers, examples include but are not limited to PVA1, PHEMA, dendritic materials and maleic anhydride substituted polymers with vinyl components to give vinyl substituted polymers including acrylate, methacrylate, allyl and maleinimide functionalities. The obtained component B based on polyfunctional polymer is defined between 1-100kDa. The substitution degree is 1-100% of available functionality. Obtained linear polymers include poly (PVAl-co-Allyl)Alyllic functionalized, poly (PVAl-co-acrylate) Acrylic functionalized, poly (PVAl-co-methacrylate) Methacrylic functionalized, poly (PVAl-co- 4-Maleimidobutyric) Maleinimido functionalized, poly (HEMA-co-Allyl) Allyl functionalized and poly (HEMA-co-Maleimido) Maleimido functionalized.

In one embodiment component B is selected from the group consisting of
- 1,3,5-triallyl-1,3,5-triazine-2,4,6(1H,3H,5H)-trione,
- tetra(ethylene glycol)diacrylate,
- poly(maleic anhydride - methyl vinyl ether) substituted with 10% 2-allyloxy ethanol, and
- poly(maleic anhydride - methyl vinyl ether) substituted with 50% 2-allyloxy ethanol.

In another embodiment the side group R3 in figure 2 is a vinyl substituent including, acrylates, methaacrylate, vinyl, allyl, unsaturated cyclic compounds, N-substituted maleimide and combinations thereof.

In another embodiment the side group R4 in Figure 2 is selected from aliphatic hydroxyl, aliphatic carboxyl and aromatic hydroxyl substituents including dopamine and p-hydroxy derivatives.

If the component B is based on a polymer the molecular weight is from 1k to 100kDa. The substitution degree ranges between 1-100% of available functionality.

The composition further comprises fibres. Fibres are added to the composition to enhance the mechanical properties. The composition including fibres forms a fibre-reinforced adhesive patch.

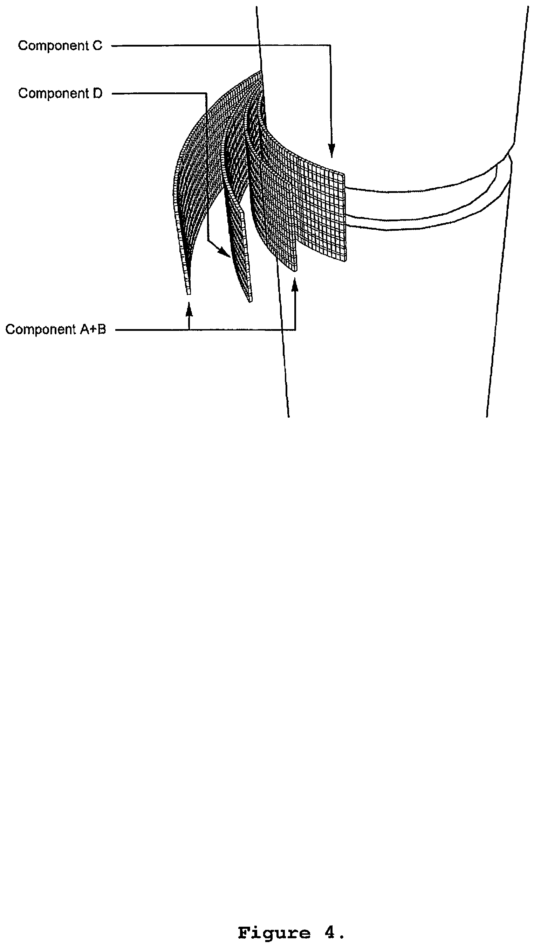

Figure 4 shows an exploded view of one embodiment with fibres in the shape of a mesh 1, surrounded on both sides by the composition 2, and applied to a fracture 3. A primer 4 is applied to the bone.
In one embodiment the fibre is a commercially available fibre, examples include but are not limited to E-glass-, S-glass-, Carbon-, UHMWPE-, Cellulose based-, collagen-, and polypropylene fibres.
In another embodiment the fibres comprise wood derived cellulose or bacterial cellulose.

In another embodiment the fibre is formed as unidirectional-, random-oriented-, 90/0 degree woven-, 45/45 degree woven-fibres. Preferably the fibres are chosen to be compliable/thin enough to follow the topology of the substrate. Number of fibres are chosen according to fracture load and surgical circumstances.
In yet one embodiment the fibre is used neat or grafted with at least one vinylic group, examples include but are not limited to acrylates, methacryaltes, allyls and unsaturated cyclic substituents including norbornenes and N-maleinimides.

There is further provided use of the composition for the manufacture of an implant for the treatment of at least one selected from a bone fracture and a bone cavity.

Many different types of bone fractures can be cured using the present invention. In one embodiment the bone fracture to be cured is a bone fracture selected from the group consisting of a compression fracture, a complete fracture, an incomplete fracture, a linear fracture, a transverse fracture, an oblique fracture, a spiral fracture, and a compacted fracture.

There is provided a method of treating a bone fracture comprising the steps applying the composition described above at the site of the fracture in the body.

The compositions according to the present invention are used for the treatment of a bone fracture of type I-III as detailed below.

### Type I - Bone Fractures

The stabilization of bone defects with the present crosslinked system through minimal invasive treatments. The cross linked system gives support to the defected bones, acting as artificial compact bones, and allowing for bone regeneration and degradation within a set timeframe. Fractures of the vertebrate bone systems are examples of Type I treatment.

The compositions according to the present invention are used together with a fibre reinforcement, examples include but are not limited to polymethylmethacrylate mesh, polypropylene mesh, fibre mesh, carbon fibre, glass fibre, biological fibres including cellulose fibres and collagen fibres. Plastic meshes examples include but are not limited to polypropylene, ethylene tetrafluoroethylene and polymethamethacylate have been used in vascular, cardiac tissue repair and cranioplasty. These meshes have been available for conventional surgical procedures.
In one embodiment fibres impregnated with the composition are applied to the fracture. This pre-impregnation of the reinforcement fibre makes the handling and application easier. A pre-impregnated fibre is abbreviated a prepreg fibre.
When using the compositions according to the present invention, a reinforcement fibre and the composition are applied to the bone where it is desired to stabilize a fracture. The composition comprising component A and B is applied, where after the composition is cured. In one embodiment, during UV curing, accelerators such as photoactivators are added. It is assumed that these additives shorten the time frame needed for the fibre reinforced patch adhesive to cure.
In one embodiment a pre-impregnated reinforcement fibre, is applied through an in-vivo delivery device such as a surgical endoscope, insertable catheter or a novel applicator device as is presently available (Method and apparatus for preparing a self-curing two component powder/liquid cement, United States Patent 4808184).

In another embodiment, component A and B premix is injected and cured in-vivo followed by the addition of impregnated fibre mesh using the above said insertable devices.

In yet another embodiment, component A and B premix is injected and impregnated with fibre mesh followed by curing, upon which each step is repeated to form multiple fibre reinforced adhesive layers.

In one embodiment the crosslinked system, the cured composition, according to the present invention and/or the reinforcement fibre, is resorbed by the body. In this embodiment the fibre composite thus will undergo dissolution over time naturally. In this embodiment the composite will eventually be replaced by growing bone without adverse effects. The patient will recover without an implant in the body after the bone fracture heals.

### Type II - Bone Cavities

The treatment of large defects of trabecular bone with highly adhesive crosslinked system, mimicking extracellular matrices, and permitting osteoblast stimulation and growth and therefore bone repair. A fracture of collum is an example of Type II treatment.

For this treatment, the component A and B are pre-mixed in the applicator. The premixed substance is injected into the cavity via the in-vivo applicator.

In one embodiment the fibres are added with the applicator.

In one embodiment the impregnated fibres are applied with a device selected from the group consisting of a surgical endoscope, an insertable catheter, and an applicator device.

In a preferred embodiment, this premix will cure on demand upon injection to form the cured composition and exhibit a porous nature that is bio-resorbable.

In yet another embodiment, this premix may contain bone growth stimulants such as osteoblasts, bone morphogenic proteins, growth hormones, cell attractants etc. This will encourage bone growth even before complete dissolution of the filler.

In a preferred embodiment, the filled cavity will be shaped and held in place with the fibre mesh mentioned in previous embodiments, fibre. The fibre mesh will be biocompatible and resorbable.

### Type III - Combined bone cavities and fractures

The treatment of large bone defects that require both trabecular and compact bone repair.

The invention is further used in various applications, not limited to bone fracture stabilization. These applications include dental fillings, maxillofacial bone fragment fixation, hip and knee replacement substitutes, small bone fracture repair, veterinary bone adhesives, post osteosarcoma repair,etc.

In one embodiment the components A and B are applied with an applicator and wherein the components A and B are mixed in the applicator upon injection.

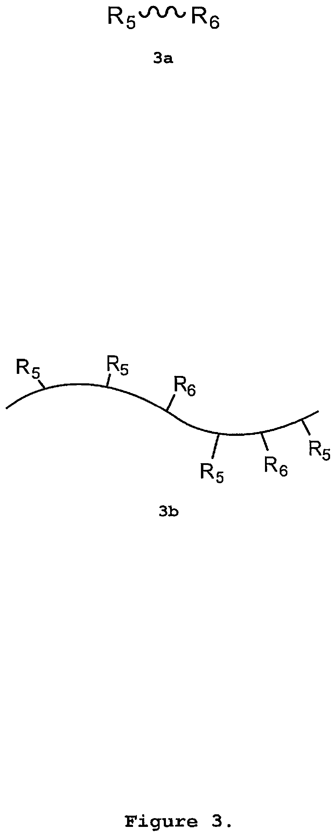

In one embodiment a primer is applied before the components A and B are applied. The primer act as an adhesion-enhancing component, to increase the adhesion between the surface and the composition. Such a primer, is in one embodiment added to the bone surface followed by a mixture of component A + B, reinforced with fibres.
The primer is described referring to Figure 3a showing an embodiment where the primer is a molecule comprising one phenolic derivative R5 and one group R6 selected from an acrylate, a methacrylate, an allyl, a vinyl, and an unsaturated cyclic ring including N-maleinimide and norbornene. Figure 3b shows an embodiment where the primer is a polymer comprising at least one group R5, wherein R5 is a phenolic derivative, and at least one group R6 wherein R6 is selected from an acrylate, a methacrylate, an allyl, a vinyl, and an unsaturated cyclic ring including N-maleinimide and norbornene.
In one embodiment R5 is a phenolic derivative.
In one embodiment R5 is selected from p-hydroxy phenyl and 3,5-dihydroxyphenyl.
In one embodiment R5 is selected from 2-hydroxyphenyl, 3-hydroxyphenyl, 4-hydroxyphenyl, 2,4-dihydroxyphenyl, 2,5-dihydroxyphenyl, 3,4-dihydroxyphenyl, 3,5-dihydroxyphenyl, and 3,4,5-trihydroxyphenyl.
In one embodiment the primer is selected from aromatic and phenolic derivatives including 4-Hydroxy-3methoxybenzaldehyde, 3,4-dihydroxyphenethylamine, para ethyl phenol, para vinyl phenol, para methacrylate phenol, Bicyclo[2.2.1]hept-2-enoic acid and poly(2- hydroxy styrene), poly(3-hydroxy styrene), poly(4-hydroxy styrene), poly(2,4-dihydroxy styrene), poly(2,5-dihydroxy styrene), poly(3,4-dihydroxy styrene), poly(3,4,5-trihydroxy styrene) and poly (3,4-dihydroxyphenethyl) acrylamide.

In yet one embodiment, the primer is synthesized by reacting hydrophilic linear polymers, examples include but are not limited to PVA1, PHEMA, Maleic anhydride substituted polymers with vinyl components to give vinyl substituted polymers including acrylate, methacrylat, allyl, maleinimide and norbornene functionalities. The obtained the primer based on linear polymer is defined between 1k-100kDa. The substitution degree is 1-99% of available functionality. Examples of synthesis routes to obtain the polymer are depicted in Fig 2. Obtained linear polymers include poly (PVAl-co-Allyl)Alyllic functionalized, poly (PVAl-co-acrylate) Acrylic functionalized, poly (PVAl-co-methacrylate) Methacrylic functionalized, poly (PVAl-co- 4-Maleimidobutyric) Maleimido functionalized, poly (HEMA-co-Allyl) Allyl functionalized and poly (HEMA-co-Maleinimido) Maleinimido functionalized.

In another embodiment the side group R5 in figure 3 is an aromatic phenol group.

In another embodiment the side group R6 is selected from vinyl subtitutens including, acrylates, methaacrylate, allyl, vinyl and unsaturated cyclic compounds including N-substituted maleinimide and combinations thereof.

In another embodiment the primer is synthesized from commercially available compounds examples include but are not limited to partly thiol or vinyl functionalized poly(para hydroxy styrene).

The primer is a dendritic polymer structure.
There is further provided an implant comprising a fibre and the composition as described above for the treatment of at least one selected from a bone fracture and a bone cavity.
In the following tables there are provided further non limiting examples of compounds which can be used as component A (Table 1), component B (Table 2), and the primer (Table 3). The abbreviations in the following table are used to denote substances used according to the present invention.

**Table 1**

| | | |
|---|---|---|
| Compontent A, Abbreviation and name of compound | 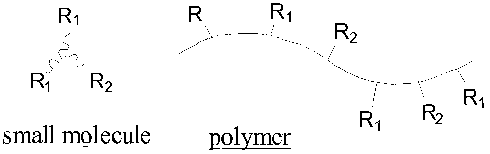 | |
| SH4 Pentaerythritol tetrakis(3-mercaptopropionate) | 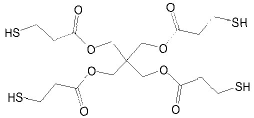 | |
| SH3 trimethylolpropane tris(3-mercaptopropionate) | 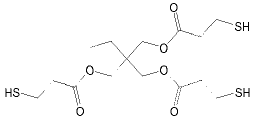 | |
| SHT3 tris[2-mercaptopropionyloxy) et hyl]isocyanurate | 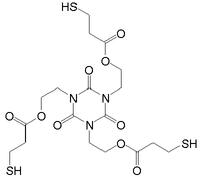 | |
| PDMS-SH (4-6% Mercaptopropyl) methylsiloxane) - dimethylsiloxane copolymer; | 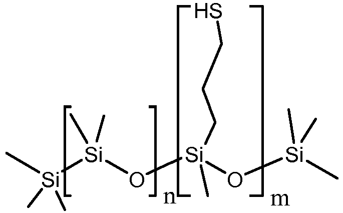 | |
| (PDMS-SH100) poly (mercaptopropyl) methylsiloxane | 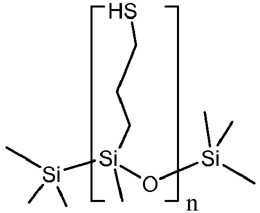 | |
| 2,2'-(Ethylenedioxy)diethane thiol | 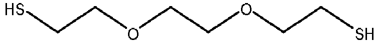 | |
| Ditiotreitol | 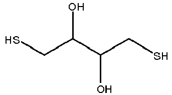 | |
| tetraethyleneglycol-bis (3-mercaptopropionate) | 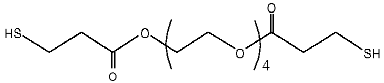 | |
| Ethyleneglycol-bis(3-mercaptopropionate) | 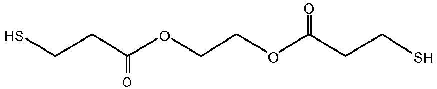 | |
| Dipentaerytritolhexakis (3-merkaptopropionate) | 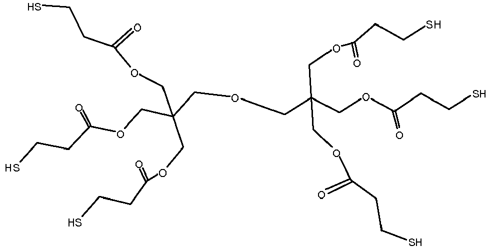 | |
| poly (maleic acid-methyl vinyl ether) functionalized with Cysteaminee, Mn: 2k-100k, Substitution degree (0 to 99%) | 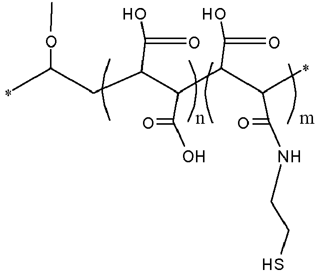 | |

**Table 2**

| | |
|---|---|
| component B, Abbreviation and name of compound | 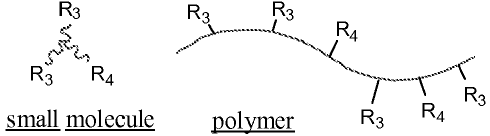 |
| TMPDE trimethylolpropane diallyl ether | 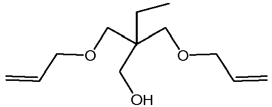 |
| 1,3,5-triallyl-1,3,5-triazine-2,4,6(1*H*,3*H*,5*H*)-trione | 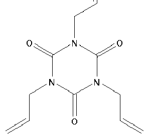 |
| TMPDE-COOH trimethylolpropane diallyl ether | 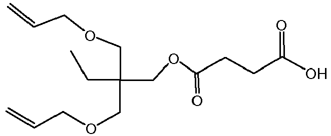 |
| diacrylate-3 tetra(ethylene glycol) diacrylate | 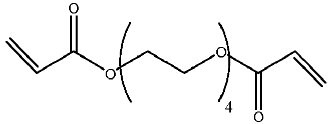 |
| diacrylate-7 poly(ethylene glycol) diacrylate | 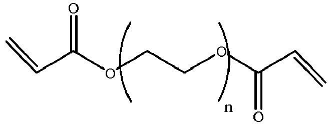 |
| Dimethacrylate-PEG 3.4k poly(ethylene glycol) dimethacrylate | 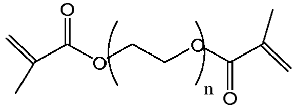 |
| Mn:3400 | |
| Allylfunctional polycaprolactone. From PCL diol, triol and tetraol Mn: 1000-100000 Da | 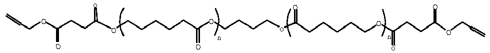 |
| poly (PVAl-co-Allyl) Alyllic functionalized poly (vinyl alcohol): Mn: 1000-500000 m= (0 to 99%) Substitution degree (SD) n(SD)= (1 to 100%) | 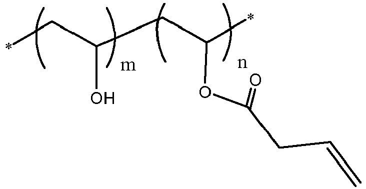 |
| poly (PVAl-co-acrylate) Acrylic functionalized poly (vinyl alcohol): Mn: 1000-500000 m= (0 to 99%) Substitution degree (SD) n(SD)= (1 to 100%) | 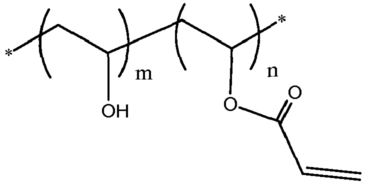 |
| poly (PVAl-co-methacrylate) Methacrylic functionalized poly (vinyl alcohol): Mn: 1000-500000 m= (0 to 99%) Substitution degree (SD) n(SD)= (1 to 100%) | 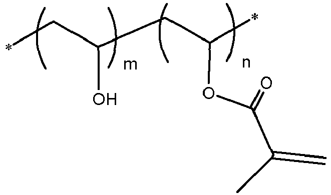 |
| poly (PVAl-co- 4-Maleimidobutyric) Maleimido functionalized poly (vinyl alcohol): Mn: 1000-500000 m= (0 to 99%) Substitution degree (SD) n(SD)= (1 to 100%) | 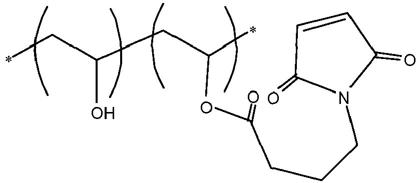 |
| poly (HEMA-co-Allyl) Allyl functionalized poly (HEMA): Mn: 1000-100000 m= (0 to 99%) Substitution degree (SD) n(SD)= (1 to 100%) | 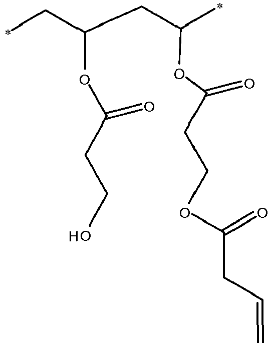 |
| poly (HEMA-co-Maleimido) Maleimido functionalized poly (HEMA) : Mn: 1000-100000 m= (0 to 99%) Substitution degree (SD) n(SD)= (1 to 100%) | 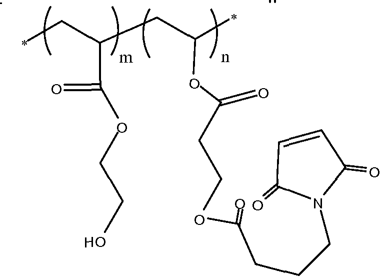 |
| tetraallyl PEO 100-1000k poly (ethylene oxid)-Allyl Mn:1000 - 10000 | 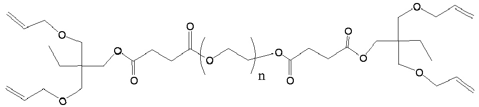 |
| Diallyl PEO 100-1000k poly(ethylene oxide) - diallyl Mn:500 - 10000 | 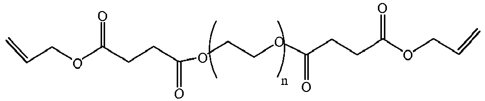 |
| poly (Maleic acid alt Methyl Vinyl Ether) functionalized with 2-allyloxyethanol Mn: 2k-100k, Substitution degree (0 to 99%) | 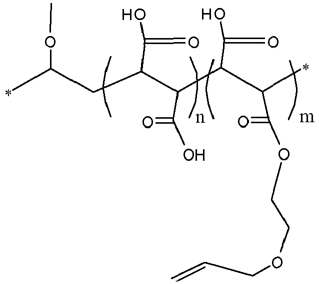 |
| poly (Maleic acid alt Methyl Vinyl Ether) functionalized with allylamine Mn: 2k-100k, Substitution degree (0 to 99%) | 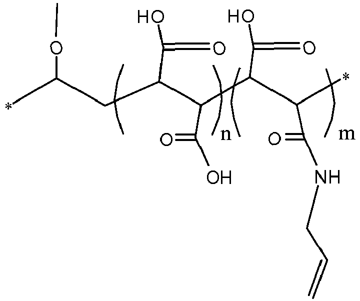 |

**Table 3**

| | |
|---|---|
| The primer, Abbreviation and name of compound | 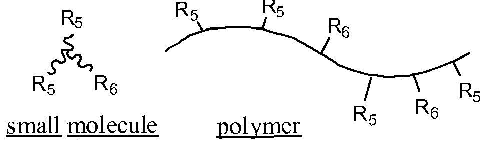 |
| poly (para-hydroxy styrene) Mn: 1-50 kDa | 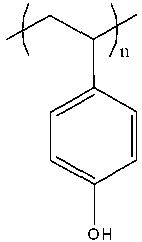 |
| poly (Para-hydroxy styrene-co-acrylate), acrylic functionalized poly (p-hydroxy styrene) : Mn: 1-50 kDa Substitution degree (SD) = (1 to 99%) | 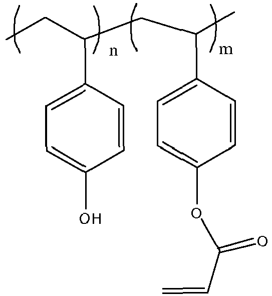 |
| poly (Para-hydroxy styrene-co-allyl), allyl functionalized poly (p-hydroxy styrene) : Mn: 1-50 kDa Substitution degree (SD) = (1 to 99%) | 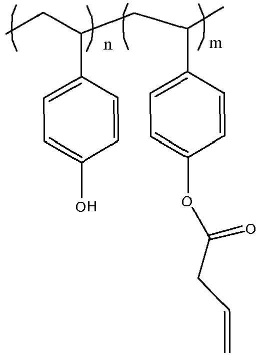 |
| poly (Para-hydroxy styrene-co-N-maleinmide), maleinimido functionalized poly (p-hydroxy styrene): Mn: 1-50 kDa Substitution degree (SD) = (1 to 99%) | 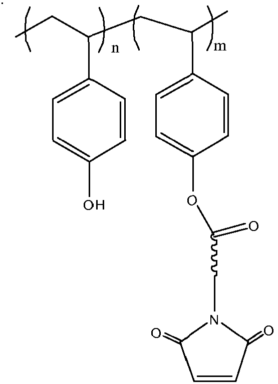 |
| poly Dopamine (poly (3,4-dihydroxy styrene)), Mn: 1-50 kDa | 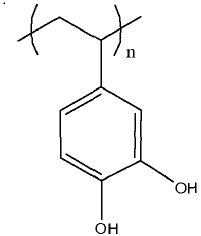 |
| Dopamine | 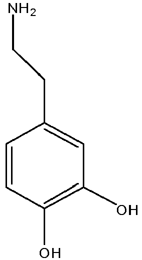 |
| Dopamine methacrylamide | 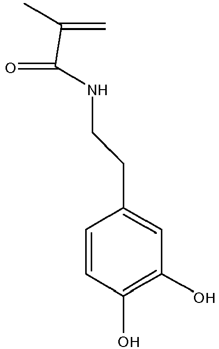 |
| Dopamine allylamide | 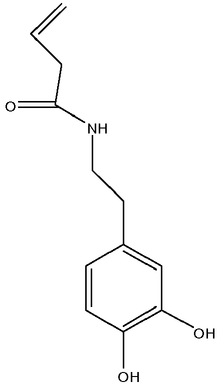 |

In one embodiment components A and B are premixed and injected/applied to the desirable designation in the body.

In another embodiment components A and B are injected/applied as separate compounds and mixed at the site in the body

In another embodiment an applicator is used to inject/apply components A and B. The components will then mix upon in-vivo injection and will be cured on demand at site

In another embodiment, the applicator will add a primer layer, the primer, followed by the premixture of components A and B.
In another embodiment, the applicator will add a primer layer, the primer, followed by the premixture of component A and B together with fibres.
In another embodiment, the applicator will add fibres, separately from the other components.
In one embodiment the composition comprising components A and B is additionally fixed in the bone by at least one screw. In this way there is provided a further way to fixate the composition in addition to adhesion. The composition to be fixed with screws comprises fibres. A primer is used. A skilled person realizes that also well-known alternatives to screws can be used such as but not limited to nails and plates. In one embodiment the composition is fixed in the bone by at least one selected from a screw and a plate. In one embodiment the implant further comprises at least one screw.
In one embodiment the composition comprises radio-opaque components, compounds detectable in the human body by x-rays, or other components to enable detection through medical imaging devices. Examples of such detection techniques include but are not limited to X-ray, and visual light.
In one embodiment the composition comprises drug molecules to further improve the healing process.
There is provided a kit for the treatment of a bone fracture comprising the composition as described above, and a primer.

In one embodiment said primer comprises at least one phenolic derivative and at least one another group selected from an acrylate, a methacrylate, an allyl, a vinyl, an unsaturated cyclic ring. The primer comprises a polymer. In one embodiment the kit further comprises at least one selected from a screw and a plate.

### Examples

Examples 1-7 describe synthesis methods for producing component A and B as well as the primer.
Examples 8-14 describe methods for applying and curing the patch.
Examples 15-16 describe cytotoxicity as well as shear strength of compositions according to the invention.

### Example 1

Synthesis of a 3 functional thiol (component A) from an allyl triazine (component B)

| | Example 1 | Moles | Grams |
|---|---|---|---|
| Allyl comp. | 1,3,5-triallyl-1,3,5-triazine-2,4,6(1*H*,3*H*,5*H*)-trione | 0.040117 | 10 |
| Thiol comp. | Thioacetic acid | 0.36105 | 27.5 |
| Reagent | AIBN | 0.0040117 | 0.659 |

1) The vinyl component, Thioacetic acid and AIBN was added to a round flask
2) Reaction was stirred for 48h in 60 C under Nitrogen atmosphere
3) Excess of Thioacetic acid was evaporated
4) Hydrolysis was conducted in 70 C in presence of THF and HCL
5) Product was purified in a column using Heptane and Ethylacetate

### Example 2

Synthesis of allyl functionalized hydrophilic poly(maleic acid alt methyl vinyl ether). This reaction yields a component B.

| | Example 2 | Moles | Grams |
|---|---|---|---|
| polymer | poly(maleic anhydride alt methyl vinyl ether), Mn=20000 | 0.000125 | 2.5 |
| Allyl comp. | 2-allyloxy ethanol | 0.01925 | 1. 97 |
| Reagent | DMAP | 0.001925 | 0.235 |
| Solvent | DMSO | - | 30mL |

1) The polymer and DMSO was added to a round flask and stirred until dissolved
2) DMAP was dissolved in DMSO and added to the round flask
3) The allyl component was diluted with DMSO and added to the round flask
4) The reaction was stirred for 24h and then quenched with water
5) The product was purified with dialysis tubes (Pore size Mw=8000) in distilled water. The dialysis water was renewed three times with neutral water followed by two times with acidic water (pH 4) and three times with neutral water
6) The products was then freeze-dried
   13C NMR characterization of the compounds reveals:

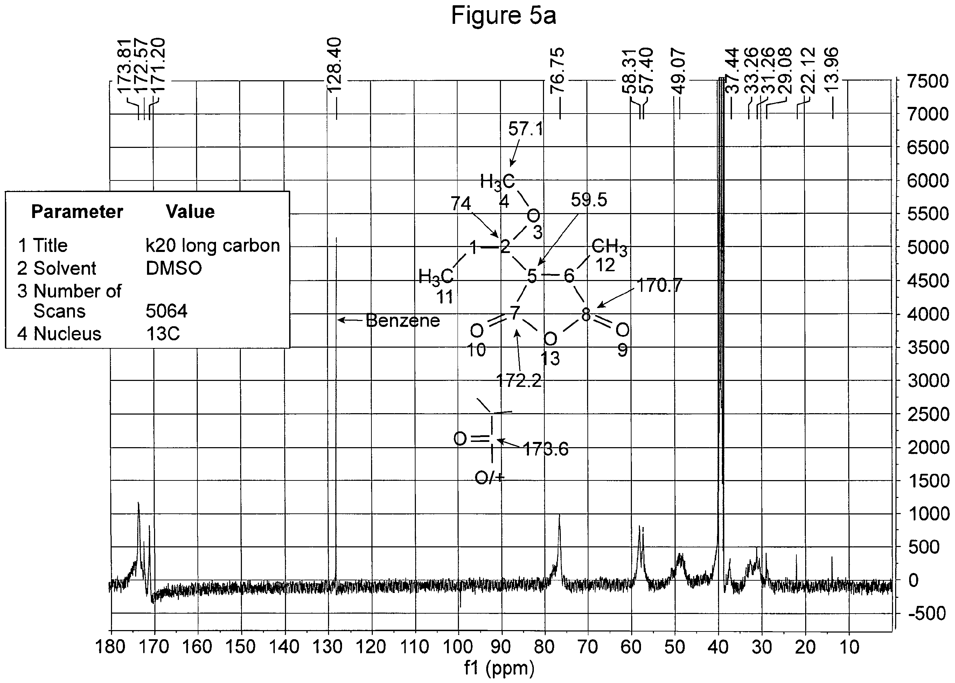

   Fig 5a: 13C NMR of poly(maleic anhydride alt methyl vinyl ether)

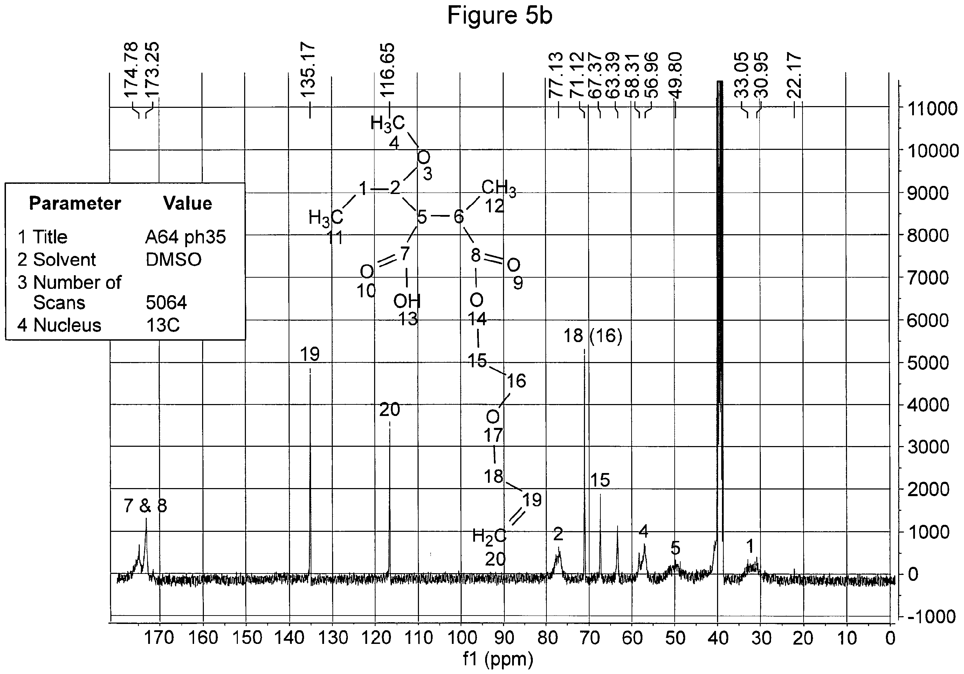

   Fig 5b: 13C NMR of allyl functionalized hydrophilic poly(maleic anhydride alt methyl vinyl ether)

### Example 3

Synthesis of a poly(para hydroxy styrene) 20% functionalized with allyl moieties. This reaction makes a compound that can be used as component B or The primer.

| | Example 3 | Moles | Grams |
|---|---|---|---|
| polymer | poly(para hydroxy styrene), Mw approx. 5000 | 0.0333 | 4 |
| Allyl | 4-pentenoic acid anhydride | 0.00666 | 1.215 |
| Reagent | DMAP | 0.000666 | 0.0815 |
| Solvent | Pyridine | - | 30ml |

1) The polymer was added to a round flask and dissolved in pyridine
2) DMAP was added and let to dissolve
3) The allyl was added slowly
4) The reaction was stirred over night
5) The reaction was quenched with water
6) Solvents were evaporated in vacuum
7) The product was dissolved in Ethanol and precipitated in cold diethyl ether
8) Product was filtered and dried

### Example 4

Synthesis of a Dopamine Methacrylamide. This reaction gives compound that can be used as The primer.

| | Example 4 | Moles | Grams |
|---|---|---|---|
| Reactant 1 | Dopamine | 0.021 | 4 |
| Reactant 2 | Methacrylic anhydride | 0.019 | 2.92 |
| Reagent | TEA | 0.023 | 2.32 |
| Solvent | Pyridine | - | 30ml |

1) Dopamine was added to a round flask and dissolved in pyridine
2) TEA was added and mixed well
3) Methacrylic anhydride was added slowly under stirring
4) The reaction was stirred over night
5) Solvents were evaporated in vacuum
6) Product was extracted and dried with Magnesium sulphate

### Example 5

Synthesis of a polyHEMA 25% functionalized with methacryates. This reaction gives a compound that can be used for component B or The primer.

| | Example 5 | Moles | Grams |
|---|---|---|---|
| Reactant 1 | polyHEMA | 0.0077 | 1 |
| Reactant 2 | Methacrylic anhydride | 0.00192 | 0.296 |
| Reagent | DMAP | 0.000196 | 0.024 |
| Solvent | Pyridine | - | 30ml |

1) polyHEMA was added to a round flask and dissolved in pyridine
2) DMAP was added and mixed well
3) Methacrylic anhydride was added slowly under stirring
4) The reaction was stirred over night
5) Solvents were evaporated in vacuum
6) Product was precipitated in cold ether

### Example 6

Synthesis of a polycaprolactone diol functionalized with allyl moieties. This reaction makes a compound that can be used as component B.

| | Example 6 | Moles | Grams |
|---|---|---|---|
| polymer | polycaprolactone diol, 3000 Da | 0.033 | 100 |
| Allyl | 4-(allyloxy)-4-oxobutanoic anhydride | 0.133 | 51.5 |
| Reagent | DMAP | 0.00666 | 0.815 |
| Reagent | Pyridine | - | 10ml |
| Solvent | Dichloromethane | - | 200ml |
| Solvent | Methanol | - | 41 |

1) Dissolve the polymer and DMAP in Pyridine and DCM
2) The allyl was added slowly
3) The reaction was stirred over night at RT
4) The reaction was precipitated in methanol
5) The precipitant was filtered and dried overnight under vacuum

### Example 7

Synthesis of a polycaprolactone diol functionalized with allyl moieties. This reaction makes a compound that can be used as component B.

| | Example 7 | Moles | Grams |
|---|---|---|---|
| polymer | polycaprolactone diol, 50000 Da | 0.002 | 100 |
| Allyl | 4-(allyloxy)-4-oxobutanoic anhydride | 0.008 | 3.09 |
| Reagent | DMAP | 0.016 | 1. 95 |
| Reagent | Pyridine | - | 10ml |
| Solvent | Dichloromethane | - | 200ml |
| Solvent | Methanol | - | 41 |

1) Dissolve the polymer and DMAP in Pyridine and DCM
2) The allyl was added slowly
3) The reaction was stirred over night at RT
4) The reaction was precipitated in methanol
5) The precipitant was filtered and dried overnight under vacuum

### Example 8

Distribution of the cured composition, with component A and component B.

| Example 8 | Structure | Mole s | Gram s |
|---|---|---|---|
| component A tetraethylene glycol bis (3-mercaptopropionat e) | 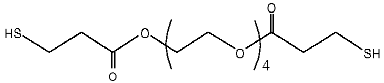 | | |
| component B poly(Maleic anhydride-methyl vinyl ether) Substituted with 50% 2-allyloxy ethanol | 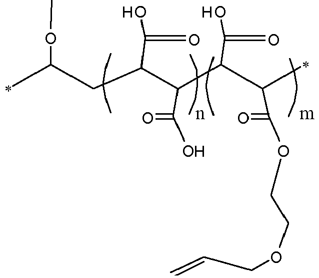 | | |
| Solvent | Ethanol/water 70/30 | | |
| Initiator | Camphorquinone | | |

1) component B was added to an empty beaker.
2) Solvent was added
3) component A was added
4) Initiator was added
5) The components and the initiator were mixed carefully in a dark environment.
6) The mixture was applied on a bone substrate
7) The mixture was allowed to cure under a UV-source at 1.66 J/cm2
8) The cured composition was allowed to reach ambient temperature before use.

### Example 9

Distribution of the cured composition, with component A and component B.

| Example 9 | | Moles | Grams |
|---|---|---|---|
| component A poly(Maleic anhydride-methyl vinyl ether) Substituted with 50% Cysteamine | 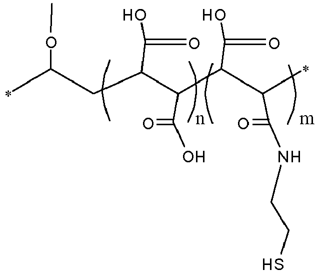 | | |
| component B poly(Maleic anhydride-methyl vinyl ether) Substituted with 50% 2-allyloxy ethanol | 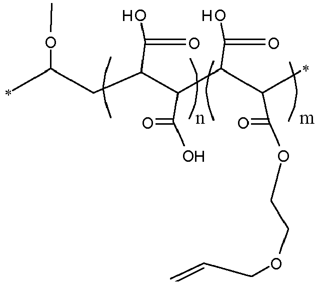 | | |
| Solvent | Ethanol/water 70/30 | | |
| Initiator | Camphorquinone | | |

1) component B was added to an empty beaker.
2) Solvent was added
3) component A was added
4) Initiator was added
5) The components and the initiator were mixed carefully in a dark environment.
6) The mixture was applied on a bone substrate
7) The mixture was allowed to cure under a UV-source at 1.66 J/cm2
8) The cured composition was allowed to reach ambient temperature before use.

### Example 10

Distribution of a cured composition, with component A and component B, to bone.

| Example 10 | | Moles | Grams |
|---|---|---|---|
| component A tris[2-mercaptopropionyloxy)ethyl] isocyanurate | 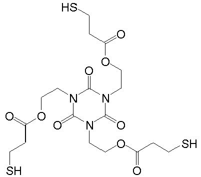 | | |
| component B1,3,5-triallyl-1,3,5-triazine-2,4,6(1*H*,3*H*,5*H*)-trione | 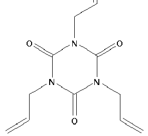 | | |

1) The vinyl component was added to an empty beaker.
2) The thiol component was added to the vinyl in a dark environment.
3) The components were mixed carefully in a dark environment.
4) The mixture was applied on a bone substrate
5) The mixture was allowed to cure under a UV-source at 1.66 J/cm2
6) The formed cured composition was allowed to reach ambient temperature before use.

Raman spectroscopy of the cured composition as above mentioned reveals following:

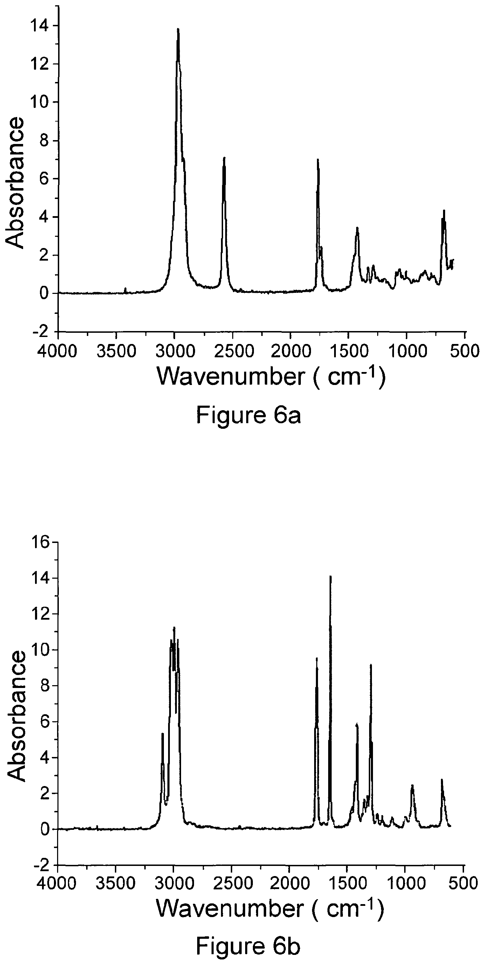

Fig 6a: component A, tris[2-mercaptopropionyloxy)ethyl] isocyanurate
Fig 6b: component B, 1,3,5-triallyl-1,3,5-triazine-2,4,6(1*H*,3*H*,5*H*)-trione

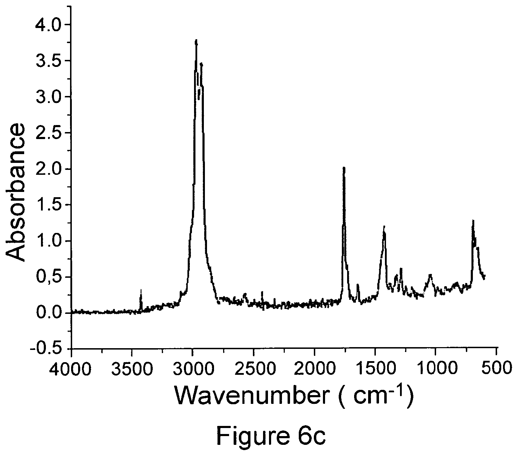

Fig 6c: Cured composition from component A and component B

### Example 11

Distribution of a cured composition, with component A component B1 and component B2, to a substrate.

| | Example 11 | Mole s | Gram s |
|---|---|---|---|
| component A tris[2-mercaptopropionyloxy)eth yl]isocyanurate | 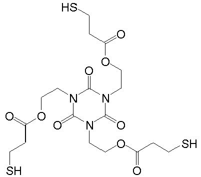 | | |
| component B1 1,3,5-triallyl-1,3,5-triazine-2,4,6(1*H*,3*H*,5*H*)-trione | 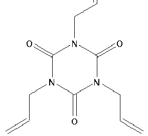 | | |
| component B2 poly(Maleic anhydride-methyl vinyl ether) Substituted with 10% 2-allyloxy ethanol | 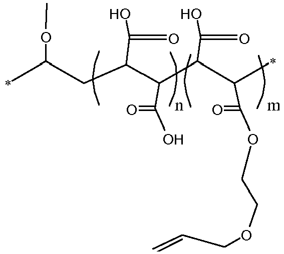 | | |
| Initiator | Camphorquinone | | |

1) The vinyl component B1 was added to an empty beaker.
2) The thiol component A was added to the vinyl in a dark environment.
3) The hydrophilic vinyl polymer, component B2 was added.
4) The components were mixed carefully in a dark environment.
5) The initiator was added and mixed well.
6) The mixture was applied on a bone substrate
7) The mixture was allowed to cure under a UV-source at 1.66 J/cm2
8) The formed cured composition was allowed to reach ambient temperature before use.

### Example 12

Distribution of a cured composition, with component A and component B, to bone.

| | Example 12 | Mole s | Gram s |
|---|---|---|---|
| component A tris[2-mercaptopropionyloxy)ethy l] isocyanurate | 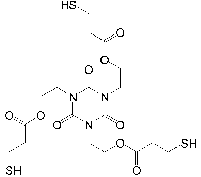 | | |
| component B tetra(ethylene glycol) diacrylate | 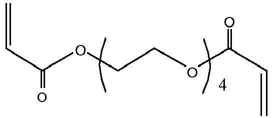 | | |

1) The component A was added to an empty beaker.
2) The component B was added to the vinyl in a dark environment.
3) The components were mixed carefully in a dark environment.
4) The mixture was applied on a bone substrate
5) The mixture was allowed to cure under a UV-source at 1.66 J/cm2
6) The formed cured composition was allowed to reach ambient temperature before use.

### Example 13

Distribution and composition of a primer, The primer, followed by a mixture of component A and component B, reinforced with the fibre.

| Example 13 | | Moles | Grams |
|---|---|---|---|
| component A tris[2-mercaptopropionyloxy)ethyl] isocyanurate | 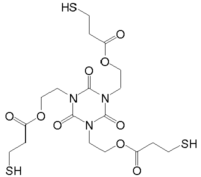 | | |
| component B 1,3,5-triallyl-1,3,5-triazine-2,4,6(1*H*,3*H*,5*H*)-trione | 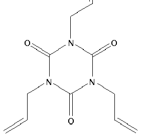 | | |
| The primer poly(para hydroxy styrene) | 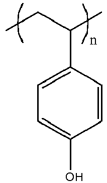 | | |
| Initiator | Camphorquinone | | |
| Solvent | Ethanol/water 70/30 | | |

1) poly(para hydroxy styrene), The primer, was dissolved in Ethanol/water 70/30 to a concentration of 25mg/ml
2) After mixing well the thin primer layer was applied around a fracture
3) the primer layer was then let to dry in approx. 1 min
4) Thereafter the vinyl, component B, was added to an empty vial.
5) The initiator was added to the vial in a dark environment and mixed carefully.
6) The thiol, component A, was then added to the vinyl and the initiator in a dark environment and mixed carefully.
7) The mixture was applied to the primer coated bone tissue as a thin layer.
8) Fibre, 4 layers of a 25g/m2 E-glass fibre veil, were then placed to on the thin mixture layer to "patch" the fracture.
9) The fibre layers were then coated with a new layer of mixture to fully wet the fibres
10) The composite patch was then cured under a UV-source at 1.66 J/cm2 to form a fibre reinforced cured composition.
11) The patch was allowed to reach ambient temperature before use.

### Example 14

Distribution and composition of a primer, The primer, followed by a mixture of component A and component B, reinforced with the fibre.

| Example 14 | structure | Moles | Grams |
|---|---|---|---|
| component A tris[2-mercaptopropionyloxy)ethyl] isocyanurate | 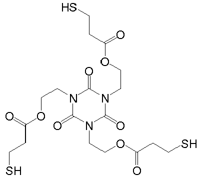 | | |
| component B 1,3,5-triallyl-1,3,5-triazine-2,4,6(1H,3H,5H)-trione | 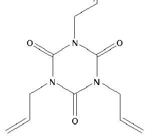 | | |
| the primer Dopamine Methacrylamide | 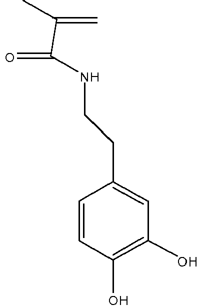 | | |
| Reagent | NaOH | | |
| Initiator | Camphorquinone | | |
| Solvent | Ethanol/water | | |

1) Dopamine Methacrylamide (AMD), the primer, was dissolved in Ethanol/water 50/50 to a concentration of 25mg/ml
2) After mixing well the thin primer layer was applied around a fracture
3) NaOH was then added to the primer to increase pH.
4) the primer layer was then let to dry in approx. 1 min
5) Thereafter the vinyl, component B, was added to an empty vial.
6) The initiator was added to the vial in a dark environment and mixed carefully.
7) The thiol, component A, was then added to the vinyl and the initiator in a dark environment and mixed carefully.
8) The mixture was applied to the primer coated bone tissue as a thin layer.
9) Fibre, 4 layers of a 25g/m2 E-glass fibre veil, were then placed to on the thin mixture layer to "patch" the fracture.
10) The fibre layers were then coated with a new layer of mixture to fully wet the fibres
11) The composite patch was then cured under a UV-source at 1.66 J/cm2 to form a fibre reinforced cured composition.
12) The patch was allowed to reach ambient temperature before use.

### Example 15

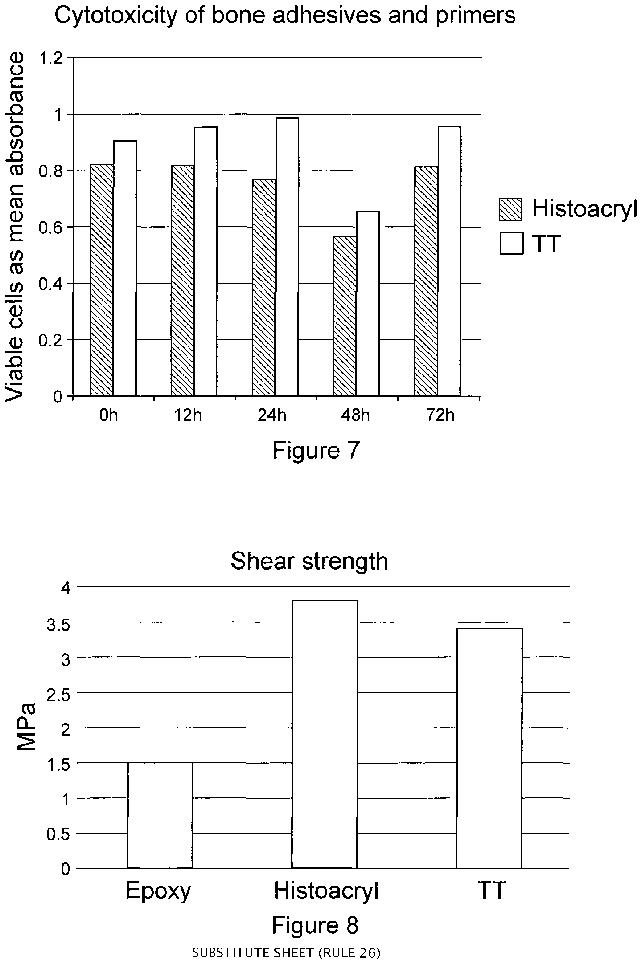

The materials to be tested were coated on glass slides (76mm x 26mm) and incubated with complete growth medium (CGM) for 24h, at a concentration of 2.5cm/ml to achieve material-CGM. MG63 osteoblast-like cells, ATCC, were cultured in Dulbecco Modified Eagle's Medium (DMEM), ATCC, containing 10% Heat inactivated Fetal Bovine Serum (FBS) and 0.5% Penicillin/Streptomycin, Sigma Aldrich, herein called as CGM. Incubation was performed at 37°C in an atmosphere of 5% CO2 and 99% humidity. CGM renewal or cell splitting was carried out once every three days. Cells were released at confluence with trypsin/EDTA and seeded on five individual 96-well plates with approximately 20000 cells/well and 200µl CGM in each well. Five replicates were performed for each type of material- CGM. The cells were allowed to attach for 12h before the CGM was replaced with 200µl of material-CGM and incubated for 0h, 12h, 24h, 48h and 72h. At each time interval each well on one plate was stained with 0.5mg/ml MTT and incubated for another 3h. After incubation the supernatant was replaced with 200µl of DMSO and the coloured solution in the wells was transferred to a new plate where absorbance was measured at 570nm in an ELISA plate reader. No toxicity could be seen for any of the tested adhesives or primers. Figure 7 shows a comparison between [1,3,5-triallyl-1,3,5-triazine-2,4,6(1*H*,3*H*,5*H*)-trione (allyl-triazine) + tris[2-mercaptopropionyloxy)ethyl]isocyanurate(thiol-triazine)], denoted as TT and the commercially available Histoacryl. It is visible that TT is more biocompatible than Histoacryl.

### Example 16

Bovine femur bones were obtained from an abattoir and split into rectangular blocks with dimensions of approximately 10x5x50 mm. Each block was then wet sanded with grain size 80 sandpaper until a smooth and even surface was achieved. A generic fracture was created by sawing each rod into two halves. The bone surface of the two halves were then prepared with a thin layer of primer and a thereafter a layer of adhesive at an approximately 8x8 mm square adjacent to the fracture. The primer-adhesive layer was then cured by exposure to UV-irradiation at a total dose of 1.66 J/cm², divided by 4 passes under a Fusion Corporation instrument with a Hg-lamp prior to the application of the fibre reinforced adhesive patch (FRAP). The FRAP bond was performed with an initial layer of adhesive followed by 6 lamina of fibres and a final top coat of adhesive. The patch was then further cured. After bonding the bone specimen were submerged in saline for 24h to mimic in vivo conditions. All mechanical tests were performed in an Instron 5568 with a 30kN load cell and with a cross head speed of 5mm/min. The tensile tests were performed by inserting two parallel metal pins through the distal ends of the specimen with wire connection to the load cell. All specimens were tested until either cohesive or adhesive failure was observed. Depending on the failure mode, either cohesive failure area or patch/bone adherent area was measured and maximum tensile strength or maximum shear strength was calculated. Testing the FRAP made from E-glass fibre reinforced [1,3,5-triallyl-1,3,5-triazine-2,4,6(1*H*,3*H*,5*H*)-trione(allyl-triazine) + tris[2-mercaptopropionyloxy)ethyl]isocyanurate(thiol-triazine)] revealed a maximum patch strength above 85MPa and a maximum shear strength when used together with a PHS primer of 3.42 MPa, compared as "TT" to the commercially available butyl-cyanoacrylate "Histoacryl" and an Industrial Epoxy in figure 8.

## Claims

1. An adhesive patch comprising
a layer comprising a primer; and
a composition provided on said primer layer, comprising the reaction product of at least one component A and at least one component B,
wherein component A comprises a compound comprising at least two thiol groups or a disulfide derivative of a compound comprising at least two thiol groups, and
wherein component B comprises vinyl reactive groups chosen from vinyl, acrylates, methacrylates, allyl and unsaturated cyclic vinyls;
wherein the primer is a dendritic polymer structure wherein the composition further comprises fibres.

2. The patch according to claim 1, wherein the fibres are in the shape of a mesh surrounded on both sides by the composition comprising at least one component A and at least one component B.

3. The patch according to any one of claims
1-2, wherein component A and B is a polymer having a molecular weight of 1 to 100 kDa.

4. The patch according to claim 1, wherein
component A is selected from the group consisting of pentaerythritol tetrakis(3-mercaptopropionate), trimethylolpropane tris(3-mercaptopropionate), tris[2-mercaptopropionyloxy)ethyl]isocyanurate, mercaptopropyl methylsiloxane-dimethylsiloxane copolymer,poly (mercaptopropyl) methylsiloxane, 2,2' (ethylenedioxy)diethanethiol, ditiotreitol, Tetraethyleneglycol-bis(3-mercaptopropionate), ethyleneglycol-bis(3-mercaptopropionate), trimethylolpropane diallylether, dipentaerytritolhexakis(3-merkaptopropionate), tetradecane-1,14-dithiol, (+ / -) -trans-1,2-bis (2-mercaptoacetamido) cyclohexane, (E) -S, S '-bis (lomercaptodecyl)-4,4'-(diazene-1,2-diyl)bis(4-cyanopentanethioate), bis(2-mercaptoethyl)sulfone, 2,5-dimercaptomethyl-1,4-dithiane, 1,4-butanediol-bis(3-mercaptopropionate), 1,16-hexadecanedithiol, undecane-1,11-dithiol, heptane-1,7-dithiol, 1,12-dimercaptododecane, octadecane-1,18-dithiol, (5-mercaptomethyl-2,4-dimethylphenyl)-methanethiol, (3-mercaptomethyl-5-methyl-phenyl)-methanetho, 1,2-benzenedimethanethiol, (4R,5R)-4,5-bis (mercaptomethyl)-2,2-dimethyl-1,3-dioxolane, 3-bis(2-mercaptoethylthio)propane, ethanethiol, aceticacidmercapto-1,2,6-hexanetriyl ester, L-1,4-dithiothretol, glycerylthioglycolate, 3,6-dioxa-1,8-octanedithiol, trimethylolpropane-tris(mercaptoacetate) 2,3-butanediol-1,4-dimercapto- pentaerythritol-tetrakis(3-mercaptopropionate), ethanethiol - 2,2',2"-nitrilotris, 2,2'-thiodiethanethiol, 1,9-nonanedithiol, 2,2'- oxydiethanethiol, and 10-decanedithiol.

5. The patch according to any one of claims
1-4, wherein component B is selected from the group consisting of trimethylolpropane diallyl ether, 1,3,5-triallyl-1,3,5-triazine-2,4,6 (1H,3H,5H) -trione, trimethylolpropane diallyl ether, poly(ethylene glycol) diacrylate, poly(ethylene glycol) dimethacrylate and poly(ethylene glycol) dimaleinimide.

6. The patch according to any one of claims
1-5, wherein the composition further comprises at least one compound selected from the group consisting of a bone growth stimulant, an osteoblast, a bone morphogenic protein, a growth hormone, a cell attractant, and a drug molecule.

7. The patch according to claim 1, wherein the fibre comprises at least one fibre selected from the group consisting of an E-glass fibre, an S-glass fibre, a carbon fibre, an UHMWPE fibre, a cellulose based fibre, a collagen fibre, and a polypropylene fibre.

8. A kit for the treatment of at least one selected from a bone fracture and a bone cavity, said kit
comprising a composition comprising the reaction product of at least one component A and at least one component B,
wherein component A comprises a compound comprising at least two thiol-groups or a disulfide derivative of a compound comprising at least two thiol groups, and
wherein component B comprises vinyl reactive groups chosen from vinyl, acrylates, methacrylates, allyl and unsaturated cyclic vinyls;
and a primer comprising a dendritic polymer structure and wherein the kit further comprises fibres.

9. The kit according to claim 8 wherein the kit further comprises at least one selected from a screw and a plate.

## Patentansprüche

1. Klebepflaster, umfassend
eine Schicht umfassend einen Primer; und
eine auf der Primerschicht bereitgestellte Zusammensetzung, die das Reaktionsprodukt von mindestens einer Komponente A und mindestens einer Komponente B umfasst,
wobei Komponente A eine Verbindung umfasst, die mindestens zwei Thiolgruppen umfasst, oder ein Disulfidderivat einer Verbindung, die mindestens zwei Thiolgruppen umfasst, und
wobei Komponente B vinylreaktive Gruppen umfasst, die aus Vinyl, Acrylaten, Methacrylaten, Allyl und ungesättigten cyclischen Vinylen ausgewählt sind;
wobei der Primer eine dendritische Polymerstruktur ist, wobei die Zusammensetzung weiter Fasern umfasst.

2. Pflaster nach Anspruch 1, wobei die Fasern die Form eines Netzgewebes haben, das auf beiden Seiten von der Zusammensetzung umgeben ist, die mindestens eine Komponente A und mindestens eine Komponente B umfasst.

3. Pflaster nach einem der Ansprüche 1-2, wobei die Komponenten A und B ein Polymer sind, die ein Molekulargewicht von 1 bis 100 kDa aufweisen.

4. Pflaster nach Anspruch 1, wobei
wobei Komponente A ausgewählt ist aus der Gruppe bestehend aus Pentaerythritoltetrakis(3-mercaptopropionat), Trimethylolpropantris(3-mercaptopropionat), Tris[2-mercaptopropionyloxy)ethyl]isocyanurat, Mercaptopropyl-Methylsiloxan-Dimethylsiloxan-Copolymer, Poly(mercaptopropyl)methylsiloxan, 2,2'-(Ethylendioxy)diethanethiol, Ditiotreitol, Tetraethylenglykol-bis(3-mercaptopropionat), Ethylenglykol-bis(3-Merkaptopropionat), Trimethylolpropandiallylether, Dipentaerytritolhexakis(3-Merkaptopropionat), Tetradecan-1,14-dithiol,(+/-)-trans-1, 2-Bis(2-mercaptoacetamido)cyclohexan, (E)-S,S'-bis(10-Mercaptodecyl)-4,4'-(Diazen-1,2-diyl) bis (4-Cyanopentanethioat), Bis(2-mercaptoethyl)sulfon, 2,5-Dimercaptomethyl-1,4-dithian, 1,4-Butandiol-bis(3-mercaptopropionat), 1,16-Hexadecandithiol, Undecan-1,11-dithiol, Heptan-1,7-dithiol, 1,12-Dimercaptododecan, Octadecan-1,18-dithiol, (5-Mercaptomethyl-2,4-Dimethyl-phenyl)-methanethiol, (3-Mercaptomethyl-5-methyl-phenyl)-methanetho, 1,2-Benzoldimethanethiol, (4R, 5R) - 4,5-Bis(mercaptomethyl)-2,2-dimethyl-1,3-dioxolan, 3-Bis(2-mercaptoethylthio)propan, Ethanthiol, Essigsäuremercapto-1,2,6-hexantriylester, L-1,4-Dithiothretol, Glycerylthioglycolat, 3,6-Dioxa-1,8-Octandithiol, Trimethylolpropan-tris(mercaptoacetat) 2,3-Butandiol-1,4-Dimercapto- entaerythritol-tetrakis(3-Mercaptopropionat), Ethanethiol - 2,2',2"-Nitrilotris, 2,2'-Tiodiethanethiol, 1,9-Nonanedithiol, 2,2'-Oxydiethanethiol und 10-Decanedithiol.

5. Pflaster nach einem der Ansprüche 1-4, wobei die Komponente B ausgewählt ist aus der Gruppe bestehend aus Trimethylolpropandiallylether, 1,3,5-Triallyl-1,3,5-triazin-2,4,6 (1H,3H,5H)-trion, Trimethylolpropandiallylether, Poly(ethylenglykol)diacrylat, Poly(ethylenglykol)dimethacrylat und Poly(ethylenglykol)dimaleinimid.

6. Pflaster nach einem der Ansprüche 1-5, wobei die Zusammensetzung weiter mindestens eine Verbindung umfasst, die aus der Gruppe ausgewählt ist bestehend aus einem Knochenwachstumsstimulans, einem Osteoblasten, einem knochenmorphogenen Protein, einem Wachstumshormon, einem Zelllockstoff und einem Arzneimittelmolekül.

7. Pflaster nach Anspruch 1, wobei die Faser mindestens eine Faser umfasst, die ausgewählt ist aus der Gruppe bestehend aus einer E-Glasfaser, einer S-Glasfaser, einer Kohlenstofffaser, einer UHMWPE-Faser, einer Faser auf Zellulosebasis, einer Kollagenfaser und einer Polypropylenfaser.

8. Kit für die Behandlung von mindestens einem, ausgewählt aus einem Knochenbruch und einer Knochenkavität, wobei das Kit
eine Zusammensetzung umfasst, die das Reaktionsprodukt von mindestens einer Komponente A und mindestens einer Komponente B umfasst,
wobei Komponente A eine Verbindung umfasst, die mindestens zwei Thiolgruppen umfasst, oder ein Disulfidderivat einer Verbindung, die mindestens zwei Thiolgruppen umfasst, und
wobei Komponente B vinylreaktive Gruppen umfasst, die aus Vinyl, Acrylaten, Methacrylaten, Allyl und ungesättigten cyclischen Vinylen ausgewählt sind;
und einen Primer, der eine dendritische Polymerstruktur umfasst, und wobei der Kit weiter Fasern umfasst.

9. Kit nach Anspruch 8, wobei das Kit weiter mindestens eine Schraube und eine Platte umfasst.

## Revendications

1. Timbre adhésif comprenant
une couche comprenant un apprêt ; et
une composition fournie sur ladite première couche, comprenant le produit de réaction d'au moins un composant A et d'au moins un composant B,
dans lequel le composant A comprend un composé comprenant au moins deux groupes thiol ou un dérivé disulfure d'un composé comprenant au moins deux groupes thiol, et
dans lequel le composant B comprend des groupes réactifs vinyle choisis à partir d'un vinyle, d'acrylates, de méthacrylates, d'un allyle et de vinyles cycliques insaturés ;
dans lequel l'apprêt est une structure polymère dendritique dans laquelle la composition comprend en outre des fibres.

2. Timbre selon la revendication 1, dans lequel les fibres sont sous la forme d'un treillis entouré sur les deux côtés par la composition comprenant au moins un composant A et au moins un composant B.

3. Timbre selon l'une quelconque des revendications 1 à 2, dans lequel le composant A et B est un polymère ayant une masse moléculaire de 1 à 100 kDa.

4. Timbre selon la revendication 1, dans lequel le composant A est sélectionné à partir du groupe constitué par le tétrakis(3-mercaptopropionate) de pentaérythritol, le tris(3-mercaptopropionate) de triméthylolpropane, le tris[2-mercaptopropionyloxy)éthyl]isocyanurate, un copolymère mercaptopropyl méthylsiloxane-diméthylsiloxane, le poly(mercaptopropyl)méthylsiloxane, le 2,2'-(éthylènedioxy)diéthanéthiol, le ditiotréitol, le tétraéthylèneglycol-bis(3-mercaptopropionate), l'éthylèneglycol-bis(3-mercaptopropionate), le diallyléther de triméthylolpropane, le dipentaérytritolhexakis(3-mercaptopropionate), le tétradécane-1,14-dithiol, le (+/-)-trans-1,2-bis(2-mercaptoacétamido)cyclohexane, le (E)-S,S'-bis(10-mercaptodécyl)-4,4'-(diazène-1,2-diyl)bis(4-cyanopentanéthioate), la bis(2-mercaptoéthyl)sulfone, le 2,5-dimercaptométhyl-1,4-dithiane, le 1,4-butanediol-bis(3-mercaptopropionate), le 1,16-hexadécanedithiol, l'undécane-1,11-dithiol, l'heptane-1,7-dithiol, le 1,12-dimercaptododécane, l'octadecane-1,18-dithiol, le (5-mercaptométhyl-2,4-diméthyl-phényl)-méthanethiol, le (3-mercaptométhyl-5-méthyl-phényl)-méthanethiol, le 1,2-benzènediméthanethiol, le (4R,5R)-4,5-bis(mercaptométhyl)-2,2-diméthyl-1,3-dioxolane, le 3-bis(2-mercaptoéthylthio)propane, l'éthanethiol, l'ester d'acide acétique-mercapto-1,2,6-hexanetriyle, le L-1,4-dithiothrétol, le glycérylthioglycolate, le 3,6-dioxa-1,8-octanedithiol, le triméthylolpropane-tris(mercaptoacétate), le 2,3-butanediol-1,4-dimercapto-pentaerythritol-tétrakis(3-mercaptopropionate), l'éthanethiol-2,2',2"-nitrilotris, le 2,2'-thiodiéthanethiol, le 1,9-nonanedithiol, le 2,2'-oxydiéthanethiol, et le 10-décanedithiol.

5. Timbre selon l'une quelconque des revendications 1 à 4, dans lequel le composant B est sélectionné à partir du groupe constitué par le diallyl éther de triméthylolpropane, la 1,3,5-triallyl-1,3,5-triazine-2,4,6(1H,3H,5H)-trione, le diallyl éther de triméthylolpropane, le diacrylate de poly(éthylène glycol), le diméthacrylate de poly(éthylène glycol) et le dimaléinimide de poly(éthylène glycol).

6. Timbre selon l'une quelconque des revendications 1 à 5, dans lequel la composition comprend en outre au moins un composé sélectionné à partir du groupe constitué par un stimulateur de croissance osseuse, un ostéoblaste, une protéine morphogénétique osseuse, une hormone de croissance, un agent attractif de cellules, et une molécule de médicament.

7. Timbre selon la revendication 1, dans lequel la fibre comprend au moins une fibre sélectionnée à partir du groupe constitué par une fibre de verre E, une fibre de verre S, une fibre de carbone, une fibre UHMWPE, une fibre à base de cellulose, une fibre de collagène, et une fibre de polypropylène.

8. Kit pour le traitement d'au moins une sélectionnée à partir d'une fracture osseuse et d'une cavité osseuse, ledit kit comprenant une composition comprenant le produit de réaction d'au moins un composant A et d'au moins un composant B, dans lequel le composant A comprend un composé comprenant au moins deux groupes thiol ou un dérivé disulfure d'un composé comprenant au moins deux groupes thiol, et dans lequel le composant B comprend des groupes réactifs vinyle choisis à partir d'un vinyle, d'acrylates, de méthacrylates, d'un allyle et de vinyles cycliques insaturés ;
et un apprêt comprenant une structure polymère dendritique et dans lequel le kit comprend en outre des fibres.

9. Kit selon la revendication 8 dans lequel le kit comprend en outre au moins une sélectionnée à partir d'une vis et d'une plaque.
